Europäisches Patentamt

(19) **European Patent Office**

Office européen des brevets

(11) Veröffentlichungsnummer **0 014 770 B1**

(12) # EUROPÄISCHE PATENTSCHRIFT

(45) Veröffentlichungstag der Patentschrift:
02.09.81

(21) Anmeldenummer: 79105372.1

(22) Anmeldetag: 24.12.79

(51) Int. Cl³ **C 07 C 15/085,** C 07 C 7/177 //
C07C13/465, C07C2/42,
C07C39/04, C07C37/08

(54) Verfahren zur Aufarbeitung des Rückcumols bei der Hock'schen Phenol-Synthese.

(30) Priorität· 19.02.79 DE 2906251

(43) Veröffentlichungstag der Anmeldung:
03.09.80 Patentblatt 80/18

(45) Bekanntmachung des Hinweises auf die Patenterteilung:
02.09.81 Patentblatt 81/35

(84) Benannte Vertragsstaaten:
AT BE FR GB IT NL SE

(56) Entgegenhaltungen:
DE-A-2 629 826
CHEMICAL ABSTRACTS, Band 78, Nr. 21, 28. Mai
1973, Zusammenfassung Nr. 135935q, Seite 352,
Columbus, Ohio US

(73) Patentinhaber: CHEMISCHE WERKE HÜLS AG,
Postfach 1320, D-4370 Marl 1 (DE)

(72) Erfinder: Ratajczak, Hans-Josef, Dr., Gersthofener
Strasse 14, D-4370 Marl (DE)

Verfahren zur Aufarbeitung des Rückcumols bei der Hock'schen Phenol-Synthese

Weltweit werden derzeit etwa 80 bis 90% des gesamten Synthesephenols nach der Hock'schen Phenol-Synthese produziert. Bei der stark exothermen Spaltreaktion des primär gebildeten Cumylhydroperoxids (65 bis 90% in Cumol) entsteht als wesentliches Nebenprodukt $\alpha$-Methylstyrol. Bei der destillativen Aufarbeitung der Reaktionsprodukte wird dieses $\alpha$-Methylstyrol sowohl zusammen mit dem Rückcumol abgetrennt und katalytisch zu Cumol hydriert als auch rein isoliert.

Die Hydrierung des $\alpha$-Methylstyrols im Rückcumol (Gehalt im allgemeinen etwa 5 bis 15%) ist jedoch vom ökonomischen Standpunkt ein absolut unbefriedigendes Vorgehen, weil dabei ein wertvolleres in ein weniger wertvolles Produkt umgewandelt wird, unter zusätzlichem Verbrauch von Wasserstoff.

Die ausschließliche Reinisolierung des $\alpha$-Methylstyrols ist ebenfalls keine optimale Lösung des Problems, weil die Abtrennung einerseits technisch recht aufwendig ist und andererseits der Bedarf an $\alpha$-Methylstyrol den dann anfallenden Mengen kaum entspricht (bevorzugte Verwendung als Comonomeres bei der Styrolpolymerisation).

Aufgabe der vorliegenden Erfindung ist daher ein Verfahren zur Aufarbeitung des Rückcumols bei der Hock'schen Phenol-Synthese, das technisch einfach durchführbar und wirtschaftlich sehr interessant ist.

Die Aufgabe wurde dadurch gelöst, daß man das Rückcumol mit einem $\alpha$-Methylstyrol-Gehalt von 1 bis 30% bei Temperaturen von 60 bis 140°C und Kontaktzeiten von 5 bis 200 Minuten mit einem Katalysator in Berührung bringt, der in an sich bekannter Weise erhalten wird, indem man getrockneten und gepulverten Bentonit (montmorillonithaltigen Ton) mit konzentrierter Schwefelsäure ohne nachfolgendes Auswaschen behandelt und die Schwefelsäure in einer Menge anwendet, die der Ionenaustauschfähigkeit des Bentonits gleichkommt oder um den Faktor 0,5 bis 1,5 abweicht und diesen Katalysator in einer Menge von 0,5 bis 50 Gewichtsprozent, bezogen auf das Reaktionsgemisch, einsetzt, wobei das $\alpha$-Methylstyrol vollständig umgesetzt wird und in hohen Ausbeuten 1-Phenyl-1.3.3-trimethylindan entsteht.

Besonders geeignet sind Katalysatoren, die gemäß der DE-C-1 069 583 hergestellt werden.

Das Cumol kann vom Reaktionsprodukt aufgrund der großen Siedepunktsdifferenz sehr leicht abgetrennt werden (z. B. durch Dünnschicht- oder Flashverdampfung).

Das 1-Phenyl-1.3.3-trimethylindan ist ein Produkt, das z. B. als Wärmeträger, Hydrauliköl oder als organisches Zwischenprodukt zur Herstellung von Anthrachinon bzw. Benzoylbenzoesäure eingesetzt werden kann.

Zur Durchführung der Dimerisierung kann der Katalysator in einem Rührgefäß vorgelegt und das $\alpha$-Methylstyrol-haltige Rückcumol hinzugegeben werden. Gegebenenfalls wird der Katalysator in wenig Cumol suspendiert.

Die Temperatur im Gefäß beträgt 60 bis 140°C. Wegen der positiven Wärmetönung der Reaktion ist es günstig, das Substrat mengengeregelt zum Katalysator zu geben, daß die Temperatur im angegebenen Bereich gehalten werden kann.

Die Katalysatormenge, bezogen auf das Reaktionsgemisch, beträgt 0,5 bis 50 Gewichtsprozent. In einer bevorzugten Ausführungsform wird der Katalysator in Staubform, insbesondere in einer Korngröße unter 100 $\mu$ eingesetzt. Geformte Kontakte (Kugeln, Strangpreßlinge) zeigen eine deutlich geringere Aktivität und Selektivität.

Überraschend bei Einsatz obiger Katalysatoren ist die große Reaktionsgeschwindigkeit bei vergleichsweise niedrigen Temperaturen. Bereits wenige Sekunden nach beendeter Zugabe vom Ausgangsgemisch kann kein $\alpha$-Methylstyrol mehr nachgewiesen werden. Die Abtrennung des festen Katalysators erfolgt in an sich bekannter Weise, beispielsweise durch Filtration oder Zentrifugieren. Der Katalysator kann ohne Nachbehandlung für weitere Ansätze verwandt werden. Die Aufarbeitung der Reaktionslösung erfolgt z. B. durch einfache Destillation oder am Dünnschichtverdampfer (gegebenenfalls im Vakuum).

Die Reaktion kann auch leicht kontinuierlich durchgeführt werden. Dabei ist besonders überraschend, daß in einem Rührgefäß mit kontinuierlichem Zu- und Ablauf auch bei sehr kurzen mittleren Verweilzeiten (etwa 5 bis 60 Minuten) vollständiger Umsatz erzielt wird. Angesichts des bekannten Verweilzeitspektrums für den einfachen Rührkessel ist dies nur durch eine außergewöhnlich hohe Reaktionsgeschwindigkeit zu erklären. Bei der kontinuierlichen Reaktionsführung wird das Produkt über eine geeignete Filtervorrichtung entnommen, so daß der Katalysator im Gefäß zurückgehalten wird. Der Stand im Gefäß wird dabei über eine entsprechende Regelung gehalten.

Die mittlere Verweilzeit liegt bei 5 bis 200 Minuten, bevorzugt bei 10 bis 100 Minuten.

Die Katalysator-Leistung (kg Rückcumol/kg Katalysator) ist sehr groß, über 10 000. Bei einem Wert von 10 000 ist noch keine Desaktivierung des Kontaktes festzustellen.

Die Aufarbeitung des Reaktionsgemisches erfolgt bei der kontinuierlichen Reaktionsführung wie bei der diskontinuierlichen Reaktionsführung.

Insgesamt stellt die vorliegende Erfindung, insbesondere die kontinuierliche Variante, ein äußerst

**0 014 770**

einfaches Verfahren zur Aufarbeitung des Rückcumols bei der Hockschen Phenol-Synthese dar, bei dem gleichzeitig ein wertvolles Zwischen- bzw. Endprodukt gewonnen wird.

Beispiele 1 bis 6

In einem 1000 ml-Dreihalskolben, der mit Rührer, Tropftrichter, Thermometer und Kühler versehen war, wurde der Katalysator, suspendiert in 100 ml Cumol, vorgelegt. Es wurden Katalysatoren der K-Serie der Süd-Chemie AG (München) eingesetzt, die gemäß der DE-C-1 069 583 hergestellt werden.

Der Kolben wurde auf Reaktionstemperatur erhitzt und 700 ml einer 10prozentigen (Gewichtsprozent) Lösung von $\alpha$-Methylstyrol in Cumol hinzugetropft. Etwa 5 Minuten nach Beendigung des Zutropfens wurde der Rührer abgestellt und nach Absitzen des Katalysators eine Probe gezogen. Nach Zugabe eines geeigneten Standards (z. B. Diphenyl) wurde die Probe durch Gaschromatographie und Hochdruckflüssigkeitschromatographie analysiert. Die HPLC-Analyse ist erforderlich, da neben dem Dimeren in kleinen Mengen noch ein Trimeres entsteht (identifiziert durch MS-Analyse), das gaschromatographisch nicht nachgewiesen werden kann.

Zur Aufarbeitung wurde der Katalysator über ein Filter abgetrennt und die Reaktionslösung destillativ aufgetrennt. Das Cumol wurde bei 0,013 bis 0,026 bar 10 bis 20 Torr) quantitativ zurückgewonnen. Die Produkte wurden im Feinvakuum destilliert (1-Phenyl-1.3.3-trimethylindan: $Kp_{0.5}$ 124 bis 126° C; Trimeres: $Kp_{15}$ 196 bis 200° C).

Die Bilanzen der Destillation stimmten mit den chromatographischen Analysen gut überein. Die Versuchsbedingungen und Ergebnisse der einzelnen Beispiele sind in der Tabelle 1 dargestellt. Im Beispiel 6 ist der Katalysator bereits zum 5. Mal eingesetzt.

Beispiele 7 bis 10

Zur kontinuierlichen Versuchsdurchführung wurde der Katalysator, suspendiert in 100 ml Cumol, in einem 1000-ml-Dreihalskolben mit Rührer und Thermometer vorgelegt. Nach Aufheizen auf Reaktionstemperatur wurde das Reaktionsgemisch (10 Gewichtsprozent $\alpha$-Methylstyrol in Cumol) mittels einer Pumpe über ein Tauchrohr mit Schliff in den Kolben gepumpt. Nach Erreichen eines bestimmten Füllstandes (800 ml) wurde der Kolben dicht verschlossen und das Produkt über eine, an ein Glasrohr mit Schliff angeschmolzene Filterkerze, die in das Reaktionsgemisch eintauchte, kontinuierlich, entsprechend der Zulaufmenge entnommen. Verstopfungen an der Filterkerze wurden nie beobachtet. Nach etwa 30 Stunden Betriebszeit wurde eine Probe genommen. Die Analytik wurde analog den Beispielen 1 bis 6 durchgeführt. Die Versuchsbedingungen und Ergebnisse sind in der Tabelle 2 zusammengestellt.

Die Ergebnisse eines Langzeitversuches sind in der Tabelle 3 aufgeführt.

3

Tabelle 1

Dimerisierung von $\alpha$-Methylstyrol im Rückcumol (diskontinuierlich)

| Beispiel | Katalysator | Menge Kat. (g) | Zutropfzeit (Min.) | Temp. (°C) | Umsatz (%) | % Ausbeute Dimeres | Trimeres |
|---|---|---|---|---|---|---|---|
| 1 | KSF/O*) | 10 | 60 | 100 | 100 | 93 | 6 |
| 2 | KSF/O | 30 | 15 | 100 | 100 | 94 | 6 |
| 3 | KSF/O | 40 | 5 | 110 | 100 | 95 | 4 |
| 4 | KSF**) | 60 | 20 | 110 | 100 | 92 | 8 |
| 5 | KSF/O | 50 | 30 | 70 | 100 | 93 | 6 |
| 6 | KSF/O | 50 | 15 | 100 | 100 | 94 | 6 |

*) Montmorillonit, $SiO_2$ ($\alpha$-Quarz), behandelt in getrockneter und gepulverter Form mit konzentrierter Schwefelsäure ohne nachfolgendes Auswaschen gemäß DE-C 10 69 583:
   innere Oberfläche 180 bis 220 $m^2$/g
   $CCl_4$-Porenvolumen in $cm^3$/g: 0,38 bis 800 Å
   Schüttgewicht: 350 bis 400 g/l.

**) Montmorillonit, $SiO_2$ ($\alpha$-Quarz), behandelt mit Schwefelsäure gemäß DE-C 10 69 583:
   innere Oberfläche 20 bis 40 $m^2$/g
   $CCl_4$-Porenvolumen in $cm^3$/g: 0,08 bis 800 Å
   Schüttgewicht: 800 bis 850 g/l.

Tabelle 2

Dimerisierung von $\alpha$-Methylstyrol im Rückcumol (kontinuierlich)

| Beispiel | Katalysator | Menge Kat. (g) | mittl. Verweilz. (Min.) | Temp. (°C) | Umsatz (%) | % Ausbeute Dimeres | Trimeres |
|---|---|---|---|---|---|---|---|
| 7 | KSF/O | 30 | 70 | 100 | 100 | 95 | 4 |
| 8 | KSF/O | 60 | 60 | 90 | 100 | 97 | 3 |
| 9 | KSF/O | 70 | 15 | 110 | 100 | 91 | 9 |
| 10 | KSF/O | 60 | 40 | 100 | 100 | 95 | 4 |

Tabelle 3

Langzeitversuch zur kontinuierlichen Dimerisierung; Bedingungen entsprechend Beispiel 10

| Laufzeit (Tage) | Kat.-Leistung (kg Rückcumol/ kg Kat.) | Umsatz (%) | % Ausbeute Dimeres | Trimeres |
|---|---|---|---|---|
| 2 | 960 | 100 | 94 | 6 |
| 4 | 1920 | 100 | 95 | 5 |
| 10 | 4800 | 100 | 95 | 4 |
| 20 | 9600 | 100 | 95 | 4 |

**0 014 770**

## Patentansprüche

1. Verfahren zur Aufarbeitung des Rückcumols bei der Hock'schen Phenol-Synthese, dadurch gekennzeichnet, daß man das Rückcumol mit einem $\alpha$-Methylstyrol-Gehalt von 1 bis 30 Gewichtsprozent bei Temperaturen von 60 bis 140°C und Kontaktzeiten von 5 bis 200 Minuten mit einem Katalysator in Berührung bringt, der in an sich bekannter Weise erhalten wird, indem man getrockneten und gepulverten Bentonit mit konzentrierter Schwefelsäure ohne ein nachfolgendes Auswaschen behandelt und die Schwefelsäure in einer Menge anwendet, die der Ionenaustauschfähigkeit des Bentonits gleichkommt oder um den Faktor 0,5 bis 1,5 abweicht und diesen Katalysator in einer Menge von 0,5 bis 50 Gewichtsprozent, bezogen auf das Reaktionsgemisch, einsetzt, wobei das $\alpha$-Methylstyrol vollständig umgesetzt wird und in hohen Ausbeuten 1-Phenyl-1.3.3-trimethylindan entsteht.

2. Verfahren nach Anspruch 1, dadurch gekennzeichnet, daß man die Umsetzung in einem Rührgefäß mit kontinuierlichem Zu- und Ablauf durchführt, wobei der Katalysator über eine geeignete Filtervorrichtung im Gefäß zurückgehalten wird.

3. Verfahren nach den Ansprüchen 1 oder 2, dadurch gekennzeichnet, daß man den Katalysator in Staubform in einer Korngröße unter 100 $\mu$ einsetzt.

## Claims

1. A method of working-up the unreacted cumene obtained in Hock's phenol synthesis, characterized in that the unreacted cumene containing $1-30\%$ by weight of $\alpha$-methylstyrene is brought into contact with a catalyst at a temperature of $60-140°C$ and for a contact period of $5-200$ minutes, the catalyst being obtained in the usual manner by treating dried and pulverized bentonite with concentrated sulphuric acid without subsequent scrubbing, the amount of sulphuric acid being $0.5-1.5$ equivalents per equivalent of bentonite based on the ion exchange capacity of the bentonite; and the amount of catalyst being $0.5-50\%$ by weight, based on the total weight of the reaction mixture, whereby the $\alpha$-methylstyrene is completely converted resulting in high yields of 1-phenyl-1.3.3.-trimethylindan.

2. The process of Claim 1, wherein the reaction is conducted in an agitated vessel with continuous feed and discharge means, and the catalyst is retained in the vessel by use of a filtering device.

3. A process of Claim 1 or 2, wherein the catalyst is in dust form of a particle size of less than 100 $\mu$.

## Revendications

1. Procédé de traitement du cumène recyclé lors de la synthèse de phénol d'après Hock, caractérisé par le fait qu'on met en contact le cumène recyclé, ayant une teneur en $\alpha$-méthylstyrène de 1 à 30 pourcent en poids pour des températures allant de 60 à 140°C et des temps de contact de 5 à 200 minutes, avec un catalyseur qu'on obtient par une méthode bien connue en traitant de la bentonite séchée et pulvérisée avec de l'acide sulfurique concentré, tout en évitant qu'un lavage consécutif n'ait lieu, et en utilisant l'acide sulfurique dans une proportion qui correspond à l'aptitude à l'échange d'ions de la bentonite ou qui varie du facteur 0,5 à 1,5 et aussi par le fait qu'on emploie ce catalyseur dans une quantité de 0,5 à 50 pourcent en poids, rapporté au mélange réactionnel, le $\alpha$-méthylstyrène étant entièrement transformé et il se forme du 1-phénol-1,3,3. trimétylindane avec un rendement élevé.

2. Procédé selon revendication 1, caractérisé par le fait qu'on effectue la transformation dans un récipient d'agitation avec entrée et sortie en continu, le catalyseur étant retenu dans le récipient au moyen d'un dispositif filtrant approprié.

3. Procédé selon l'une quelconque des revendications 1 ou 2, caractérisé par le fait qu'on utilise le catalyseur sous forme pulvérulente à une granulométrie inférieure à 100 $\mu$.

5